# FASCICULE DE BREVET EUROPEEN

(11) **EP 0 562 208 B1**
(45) Date de publication et mention de la délivrance du brevet: **10.04.1996**
(21) Numéro de dépôt: 92500170.3
(22) Date de dépôt: 23.12.1992
(51) Int. Cl.: A61B 5/0428, A61N 1/22, A61N 1/32

(54) **Plastron destiné au positionnement d'électrodes ECG**
Brustschild zur Positionierung von ECG-Elektroden
Chest-piece for positioning ECG electrodes

(30) Priorité: 27.12.1991
(43) Date de publication de la demande: 29.09.1993
(73) Titulaire: Hernandez Herrero, Juan, E-08034 Barcelona (ES)
(72) Inventeur: Hernandez Herrero, Juan, E-08034 Barcelona (ES)
(74) Mandataire: Manresa Val, Manuel

(56) Documents cités:
- EP-A- 0 242 546
- US-A- 3 490 439
- US-A- 4 308 870
- 9TH ANNUAL CONFERENCE OF THE IEEE/EMB SOCIETY 16 Novembre 1987, BOSTON, MA (US) pages 178 - 181 A. SIPPENS GROENEWEGEN ET AL. 'A Radiotransparent Carbon Electrode Array for Body Surface Mapping...'

## Description

### Domaine de l'invention

L'objet de la présente invention concerne un plastron spécialement étudié pour des électrodes de dérivations précordiales de l'électro-cardiogramme.

### Antécédents de l'invention

La réalisation d'un électro-cardiogramme, dans la pratique quotidienne, se heurte à l'inconvénient de la mise en place des électrodes sur le thorax pour enregistrer les dénommées "dérivations précordiales".

La pratique d'un électro-cardiogramme consiste à enregistrer, depuis la surface externe de l'organisme, l'activité électrique du coeur.

L'enregistrement se fait par un appareil, l'électro-cardiographe, qui recueille l'activité électrique cardiaque à travers de câbles, qui sont branchés à des électrodes d'enregistrement, lesquels entrent en contact avec la surface corporelle.

Suivant le point de la surface corporelle sur lequel l'électrode d'enregistrement est posé, les ondes électriques seront observées avec une ou autre morphologie, ce qui constitue une des bases de l'électro-cardiographie.

Dans l'électro-cardiogramme conventionnel, il y a douze dérivations établies universellement selon le point d'observation, suivant la mise en place des électrodes. Six de celles-ci sont les dénommées "dérivations de membres" et les autres six sont les dénommées "dérivations précordiales".

Les dérivations de membres ont besoin de la mise en place des électrodes pour leur enregistrement sur les bras et les jambes, à quelque point de leur longueur, ou bien sur le tronc à proximité des racines des bras et des jambes.

Les dérivations précordiales sont respectivement dénommées v1, v2, v3, v4, v5 et v6, et elles ont des points d'emplacement précis, à savoir:
v1 sur le quatrième espace intercostal droit, près du sternum.
v2 sur le quatrième espace intercostal gauche, près du sternum.
v3 équidistante entre v2 et v4.
v4 sur le cinquième espace intercostal gauche sur la ligne claviculaire du milieu.
v5 sur le cinquième espace intercostal gauche sur la ligne axillaire avant
v6 sur le cinquième espace intercostal gauche sur la ligne axillaire moyenne.

Pour que l'enregistrement soit clair et puisse être correctement interprété il faut des connections et des isolements corrects dans l'appareil d'enregistrement, une préparation appropriée de la surface corporelle sur le point de contact des électrodes, et leur fixation parfaite pour qu'ils ne bougent pas pendant l'enregistrement.

L'enregistrement des dérivations de membres ne pose généralement pas de problèmes, car, premièrement, l'immobilité de l'électrode d'enregistrement est facile d'obtenir avec un quelconque des systèmes actuels et, deuxièmement, l'image électrique ne varie pas trop même si l'électrode d'enregistrement de chaque membre est situé sur un ou autre point de sa longueur, les images étant comparables aussi bien si l'enregistrement a été fait de la racine du membre comme s'il a été fait sur quelque point du trajet jusqu'à l'extrémité distale.

Il n'en est pas de même pour les dérivations précordiales qui posent chaque jour des problèmes pour leur enregistrement, avec les conséquences qui seront énoncées plus bas.

En ce qui concerne la fixation des électrodes sur le thorax, généralement, on essaie de faire tenir en place l'électrode d'enregistrement par l'intermédiaire d'une ventouse, et c'est courant que des problèmes surgissent pour diverses causes, qui sont, fondamentalement:
A) Perte de la capacité de succion de la ventouse, ce qui empêche sa stabilité au point désiré.
B) Excès de succion de la ventouse, ce qui provoque des irritations cutanées, quoiqu'elles sont normalement légères.
C) Impossibilité de maintenir l'électrode stable, à cause des mouvements respiratoires de la personne explorée.
D) Impossibilité de le maintenir stable à cause de duvet du thorax qui rend la fixation difficile.
E) Impossibilité de la maintenir stable pour des anomalies thoraciques.

Très souvent, la difficulté que le duvet du thorax représente est si grande qu'elle oblige à raser le thorax de la personne explorée, au moins sur la bande de placement des électrodes précordiaux.

La difficulté de fixation de l'électrode est un problème très fréquent et comporte une perte importante de temps pour réaliser le traçage et, même ainsi, souvent on n'obtient pas une stabilité suffisante, ce qui représente un traçage ayant une qualité faible pour manque de netteté de l'enregistrement.

Par ailleurs, l'exactitude de la mise en place des électrodes d'enregistrement pour les précordiales est essentielle pour l'étude ultérieure du traçage, car les images électro-cardiographiques fournissent des informations précieuses sur le parcours des vecteurs électriques du coeur dans chaque impulsion du coeur, et la direction, sens, magnitude en voltage et durée en temps de chaque onde enregistrée deviennent des données fondamentales pour le diagnostic.

L'emplacement précis de chaque électrode précordial est fondamental pour l'interprétation des images qui sont recueillies à travers lui et sa mise en place inadéquate sur le thorax peut entraîner des erreurs de diagnostic importantes, aussi bien parce qu'elles cachent des pathologies existantes, que parce qu'elles semblent en montrer d'autres inexistantes.

Egalement, lorsqu'on fait des traçages successifs à une même personne, les images des dérivations de membres sont toujours comparables même si les électrodes n'ont pas été placés sur le même point chaque fois, mais pour que les images des précordiales puissent être comparables il faut être sûr qu'elles ont été recueillies du même point, car de petites différences d'emplacement peuvent donner lieu à des écarts dans l'enregistrement, et le médecin doute souvent si le patient a eu des variations de son état clinique ou si les enregistrements précordiaux ne sont pas fait sur exactement le même point.

Lorsqu'un patient est soumis à des explorations successives à un certain établissement et, notamment, lorsqu'il est hospitalisé dans une salle, unité de cardiologie ou de soins intensifs, il faut très souvent faire des électro-cardiogrammes en séries, à des intervalles de quelques jours les uns des autres, chaque jour ou même plusieurs fois par jour, pour comparer son évolution, et dans ce cas, plus que jamais, il faut avoir la certitude que la mise en place des dérivations précordiales est toujours la même, car de petits écarts dans la mise en place des électrodes peuvent produire les erreurs ci-dessus signalées. On essaie de le résoudre, quelquefois, en peignant sur la poitrine du patient des points qui servent de repère pour savoir où ils ont été placés la première fois et les remettre toujours à la même place, mais cette solution n'est pas bonne à cause du problème esthétique qu'elle représente et parce qu'après, la transpiration et la toilette qu'il faut faire au malade, effacent les points marqués. A. Sippens Groenewegen et al. (9TH ANNUAL CONFERENCE OF THE IEEE/EMB SOCIETY, 16 Novembre 1987, Boston, US, pg 178 - 181) décrivent un dispositif selon le préambule de la revendication 1.

Nous proposons un système d'enregistrement, objet de la présent demande de dépôt de brevet qui offre une solution définitive des problèmes énoncés et, sans entraîner de gênes pour le malade, il résout le problème de la fixation et garantit l'emplacement exact des enregistrements, avec les avantages qui en découlent.

### Description d'une action préférée de l'invention

Afin de rendre l'explication plus facile, il est annexé à la présente description une page de dessins sur laquelle il a été représenté un cas pratique de mise en oeuvre, qui n'est cité qu'à titre d'exemple non limitatif de l'étendue du présent Brevet d'Invention.

Sur ces dessins:

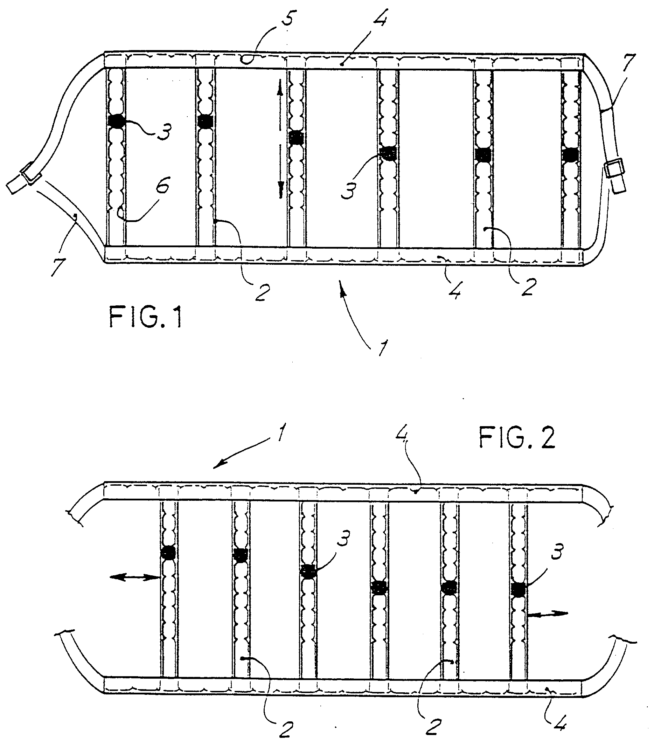

Les figures 1 et 2 illustrent le plastron préparé, respectivement, pour un individu ayant un thorax large et pour un autre individu au thorax plus étroit.

Il consiste en un plastron -1- en matériau suffisamment souple pour s'adapter au thorax de la personne explorée. Il a plusieurs bandes -2- disposées longitudinalement, chacune d'elles héberge un électrode -3- pour l'enregistrement d'une dérivation précordiale. Ce plastron -1-, qui peut avoir une surface à peu près rectangulaire ou avec des variations de format que l'on souhaite lui donner, a des glissières transversales -4- à leurs bords supérieur et/ou inférieur qui servent pour qu'à travers lui(elle) les bandes -2- disposées longitudinalement qui sont parallèles au plus grand axe du sternum glissent.

Dans les bandes longitudinales -2-, normalement six, une pour chaque dérivation précordiale, les électrodes -3- glissent de haut en bas ou de bas en haut, pour enregistrer les précordiales.

Aussi bien pour le déplacement vertical des électrodes -3- que pour le déplacement transversal des bandes -2- qui les contiennent, les glissières sur lesquelles ils glissent permettent leur mise en place exactement là ou l'on veut, et on dispose de saillies et d'entailles -5-, -6- pour les fixer, dès que l'on a choisi le point où on veut les situer.

Lorsqu'on déplace les bandes -2- qui contiennent les électrodes -3- vers la droite ou vers la gauche, on les place près des bords du sternum sur la ligne correspondant à v3 (non représentée) sur la ligne semi-claviculaire, sur les lignes axillaires avants ou du milieu, ou au point que l'on désire en direction transversale.

Lorsqu'on déplace chaque électrode -3- vers le haut ou vers le bas de la bande qui le contient, en direction cranio-caudal dans un quelconque de ses deux sens, on le situe sur un ou autre espace intercostal ou sur une côte.

Ainsi, avec la combinaison des deux déplacements transversal et longitudinal, on peut situer chaque électrode -3- sur le point précis pour l'enregistrement de la dérivation lui correspondant et on s'adapte ainsi aux dimensions et caractéristiques du thorax de chaque personne.

S'il faut explorer un patient plusieurs fois de suite, on peut conserver le plastron du premier enregistrement sans changer la position des électrodes, et l'usage de "son" plastron garantit que l'emplacement des électrodes sera toujours la même.

On peut penser qu'une source de possible erreurs sera que le plastron soit placé sur des points différents chaque fois, mais c'est facile de résoudre, car on peut faire une marque, par exemple l'angle sternum-costal, sur le plastron même qui servira de repère pour les prochaines fois ou bien le plastron même peut déjà avoir une entaille à ce niveau ou un autre repère anatomique, en garantissant son emplacement parfait les autres fois.

Le plastron aura des rubans -7- ou un autre moyen de fixation qui, sans représenter de gêne pour la personne explorée, permettra de le conserver ferme sur le thorax sans qu'il ne glisse durant l'enregistrement.

Le plastron -1- sera fait en un matériau suffisamment souple pour s'adapter à la configuration du thorax de chaque personne et faire un bon contact sur toute sa surface.

Chez certains patients, le personnel de soin souhaite explorer d'autres zones du thorax. Un cas typique est la présence ou le soupçon d'infarctus affectant la face arrière du coeur, les électrodes -3- étant donc pour ce portés à la partie arrière de l'hémithorax gauche. D'autres fois on souhaite enregistrer des dérivations de l'hémithorax droit, par exemple, lorsque l'on soupçonne un infarctus du ventricule droit. Pour ces cas on peut dessiner des plastrons spéciaux, plus larges et ayant un plus grand nombre de bandes porteuses d'électrodes.

Avec ce qui a été exposé on résout définitivement les deux problèmes posés au début de l'exposé: d'une part l'enregistrement difficile pour manque de fixation des électrodes, et d'autre part la non comparabilité des enregistrements successifs parce que les électrodes n'ont pas été placés exactement à la même place.

## Revendications

1. Dispositif de mise en place d'électrodes pour l'enregistrement de dérivations précordiales électro-cardiographiques, ledit dispositif étant souple pour s'accommoder à l'anatomie thoracique de la personne à explorer et consistant en une pluralité de bandes longitudinales parallèles (2) susceptibles d'être mises en place parallèlement au plus grand axe du sternum, lesdites bandes longitudinales parallèles étant susceptibles d'héberger des électrodes précordiaux (3) **caractérisé** en ce que le dispositif consiste en un plastron (1), en ce que lesdites bandes longitudinales parallèles (2) sont susceptibles de glisser à droite et à gauche de glissières transversales (4), supérieure et inférieure, du plastron (1) même et en ce que les électrodes précordiaux (3) sont susceptibles de glisser dans les bandes longitudinales parallèles, le long de celles-ci.

2. Dispositif selon la revendication 1, caractérisé en ce que, aussi bien les bandes longitudinales parallèles (2) que les glissières transversales (4) comptent sur une série d'entailles ou de saillies (5,6) pour fixer les diverses positions de l'électrode sur la propre bande et de fixer les bandes sur ces glissières.

3. Dispositif selon les revendications précédentes, caractérisé en ce qu'il porte des rubans (7) ou un autre moyen similaire pour fixer le plastron même sur le thorax de la personne explorée.

## Claims

1. Device for positioning electrodes to record precordial electrocardiographic leads, said device being flexible to suit the thoracic anatomy of the person to be explored and consisting in a plurality of parallel longitudinal bands (2) susceptible of being positioned parallel to the major axis of the sternum, said parallel longitudinal bands being susceptible to house precordial electrodes (3), characterized in that the device consists in a breastplate (1), in which said parallel longitudinal bands (2) are susceptible to slide to the right and to the left of upper and lower cross-guides (4) of the breastplate (1) itself and in that the precordial electrodes (3) are susceptible to slide within the parallel longitudinal bands, along them.

2. Device according to the claim 1, characterized in that, as well the parallel longitudinal bands (2) as the cross-guides (4) have a series of notches or protrusions (5,6) to set the different positions of the electrode on the band itself and to fasten the bands on said guides.

3. Device according to the preceding claims, characterized in having ribbons (7) or another similar means to fasten the breastplate itself on the thorax of the explored person.

## Patentansprüche

1. Apparat zum Einsetzen von Elektroden zur Aufnahme von präkordialen elektrokardiographischen Ableitungen, wobei dieser Apparat flexibel ist und sich an die Brustkorbanatomie der zu untersuchenden Person anpassen läßt, bestehend aus einer Vielzahl paralleler Längsbänder (2), die sich parallel zur Hauptachse des Brustbeines einsetzen lassen, und die präkordiale Elektroden (3) aufnehmen können, dadurch gekennzeichnet, daß er aus einem Latz (1) besteht und die genannten parallelen Längsbänder (2) in der Lage sind, auf den unteren und oberen Querführungen (4) nach links und rechts zu gleiten, und die präkordialen Elektroden (3) auf den parallelen Längsbändern gleiten können.

2. Apparat gemäß Anspruch (1), dadurch gekennzeichnet daß sowohl die parallelen Längsbänder (2) als auch die Querführungen (4) eine Reihe von Einschnitten bzw. Vorsprüngen (5,6) zur Bestimmung mehrerer Stellungen der Elektrode auf dem Längsband selbst sowie zur Befestigung der Langsbänder auf diesen Führungen aufweisen.

3. Apparat gemaß vorstehenden Ansprüche, dadurch gekennzeichnet, daß er Riemen (7) bzw. andere ähnliche Mittel zur Befestigung des Latzes am Brustkorb der untersuchten Person aufweist.
